# EUROPEAN PATENT APPLICATION

(11) **EP 4 840 416 A1**
(43) Date of publication of application: **18.11.2026**
(21) Application number: 25175981.7
(22) Date of filing: 13.05.2025
(51) Int. Cl.: A61B 18/20, A61N 5/06

(54) **ELECTRIC DEVICE AND A METHOD FOR CONTROLLING THE ELECTRIC DEVICE FOR TREATMENT OF A SKIN**

(71) Applicant: 12M Invent GmbH, 91245 Simmelsdorf (DE)
(72) Inventor: SEEFELDT, Kai, 91245 Simmelsdorf (DE)
(74) Representative: Grünecker Patent- und Rechtsanwälte PartG mbB

(57) **Abstract**

The present disclosures relates to an electric device (10) for attachment on a skin (200). The device (10) comprises a support structure (110) with an optical system (120) for illuminating the skin, wherein an exit aperture (121) of the optical system (120) is arranged on an inner wall (112) of the support structure (110); a sealing (130) arranged on the support structure (110), the sealing (130) surrounding the exit aperture, wherein the sealing (130), when contacting the skin, forms a sealed chamber (300) comprising the inner wall (112); a pressure control system (140) in fluid connection with the sealed chamber (300) for generating a differential pressure in the sealed chamber (300) compared to outside the sealed chamber; and a release unit (400) configured to activate the optical system (120) based on the differential pressure in the sealed chamber (300).

## Description

The invention relates to an electric device and a method for controlling the electric device for treatment of a skin.

Light can be used in non-invasive technologies that apply electrical energy via an optical system to the body to stimulate physiological responses in the skin. The optical system for stimulating the skin is typically a designed to emit specific wavelengths of light that can interact with skin tissues for therapeutic, cosmetic, or diagnostic purposes. For example, the tissue may be heated by the light.

The device must comply with medical device standards and may need to include safety features in view of the use of high intensity optical systems.

The object of the invention is to further improve an electric device comprising an optical system. In particular, the objection is to increase security.

This object is solved by the independent claims. Advantageous embodiments are solved by the dependent claims.

According to a general aspect, an electric device comprises a release unit. The release unit may activate components such as the optical system based on a differential pressure. In particular, a negative pressure in a chamber surrounding the light source of the optical system facilitates that the light source is fixed attached to the skin. Additional, a differential pressure in the chamber relative to an ambient pressure facilitates to decide if the device connected in a sealed manner to the skin or not.

Notably, the optical system can be used to apply electrical energy by electric fields. Electric fields can be used in non-invasive technologies that apply electrical energy to the body via external pads to stimulate physiological responses in underlying tissues. Depending on the wavelength, different effects may be achieved.

For example, RF (Radiofrequency) plates can be used in aesthetic and therapeutic medical treatments to deliver controlled heat to deeper layers of tissue. This is achieved by applying highfrequency alternating current through pads (e.g. p plate-shaped electrodes), which generates heat via tissue resistance. The resulting thermal effect stimulates collagen production, tightens skin, and can reduce fat in localized areas, making RF plates common in non-invasive anti-aging and body contouring procedures. In physical therapy, RF can also promote circulation and tissue repair by increasing blood flow and metabolic activity in targeted areas.

Further, EMS (Electrical Muscle Stimulation) plates, deliver electrical impulses to muscles via pads, e.g. flat electrodes, typically in the form of adhesive or rigid plates. These impulses mimic the action potentials from the central nervous system, causing muscles to contract. EMS plates are widely used in sports training, rehabilitation, and muscle re-education-helping strengthen weakened muscles, maintain muscle mass during inactivity, and improve endurance. In cosmetic applications, EMS plates can also be used to tone and firm specific muscle groups, such as abdominal or facial muscles.

For both, RF and EMS plates have in common is that both are conductive applicators to deliver energy across the skin surface, and both aim to target specific tissues beneath the skin without penetration of the skin by the pads - whether to stimulate muscles (EMS) or to induce thermal effects (RF).

Applicators may be bipolar or monopolar. Bipolar applicators offer the key advantage of localized and controlled energy delivery by integrating both the active and return electrodes within a single applicator. This configuration ensures that the electric current or radiofrequency energy flows only through the tissue directly between the two electrodes, resulting in precise targeting of specific areas without affecting surrounding or deeper regions. As a result, bipolar systems are often safer, more comfortable, and require lower power levels compared to monopolar setups, reducing the risk of unintended tissue stimulation or burns. Their ease of application and self-contained design make them particularly suitable for portable devices, aesthetic treatments, and focused therapeutic interventions, such as in facial EMS, RF skin tightening, or localized muscle activation.

A further object of the invention is to further improve an electric device comprising a bipolar applicator. In particular, the objection is to more precisely control the area that can be stimulated with the applicator. A further object is to increase the flexibility to stimulate various regions.

The invention will now be described in more detail and by way of example using advantageous embodiments and with reference to the drawings. The embodiments described are possible configurations in which the individual features described above can be provided independently of one another or can be omitted.

In the figures,
**Figure 1** is a schematic of an electric device according to an example of the present description.
**Figure 2** is the electric device of figure 1 after placing the electric device on a skin.
**Figure 3** is the electric device of figure 1 after attaching to the skin.
**Figure 4** is a schematic of the electric device according to figure 1 seen from above.
**Figure 5** is a block diagram of the electric device according to an example.
**Figure 6** is a schematic block diagram of the controller of the electric device.

A first aspect of the present disclosure relates to an electric device for attachment to a skin. The device can be physically secured to the skin surface to maintain consistent contact during use. This facilitates effective and safe transmission of electrical energy (for example by a light source, EMS or RF treatments), ensuring the device stays in the correct position and delivers energy evenly.

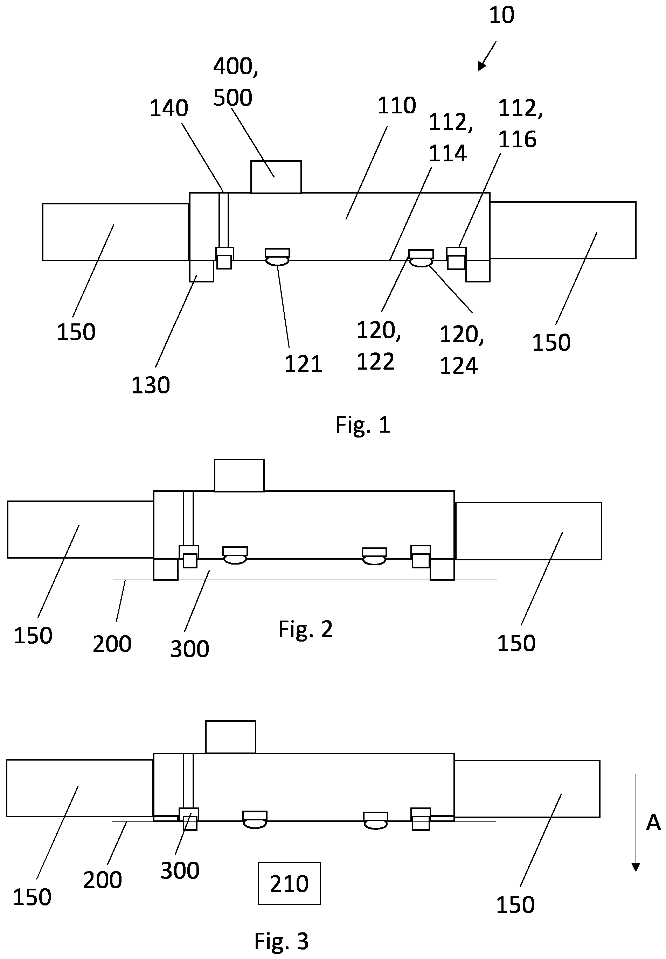

According to the first aspect, the device comprises a support structure with an optical system for illuminating the skin as for example shown in Fig. 1 to 3. In particular, the optical system 120 comprises an exit aperture 121 that is arranged on an inner wall 112 of the support structure 110.

As used the exit aperture refers to the opening through which light exits an optical system, such as a light source device, e.g. laser or an light emitting diode (LED). It defines the final boundary of the optical path before the light reaches the target - in this case, this in particular the skin.

Additionally or alternatively, the optical system may comprise an electrode system 150 for contacting the skin as for example shown in Figs. 1 to 4.

The support structure comprises at least two opposing faces, wherein one is herein referred as an inner wall designed for direct attachment to the skin, for example made of biocompatible materials and shaped for ergonomic, stable contact. This inner wall comprises at least one exit aperture and may incorporate additionally integrated components like sensors or electrodes. The outer wall, positioned opposite the inner wall, functions as a protective and structural element housing external interfaces or support features. The support structure arranged in the space between the walls can comprises the optical system, electronics, and components for controlling a differential pressure a sealed chamber.

According to the first aspect, the device comprises a sealing 130 that is arranged on the support structure 110. The sealing 130 surrounds the exit aperture, wherein the sealing 130, when contacting the skin, forms the sealed chamber 300 comprising the inner wall 112. This facilitates that eh the

Additionally or alternatively, the optical system may comprising the electrode system 150 may surround the sealing 130.

As used herein, the sealing refers to a structural or material feature used to prevent the passage of fluids, in particular air, and light between adjoining parts. In the context of the support structures, the sealing facilitates that exit aperture arranged on the inner wall is sealed from ambient.

According to the first aspect, the device comprises a pressure control system 140 in fluid connection with the sealed chamber 300 for generating a differential pressure in the sealed chamber 300 compared to outside the sealed chamber.

As used herein, the pressure control system is a mechanism, for example an air pump, designed to regulate a desired pressure level, herein referred to a as differential pressure, within a defined space, herein the sealed chamber. If the mechanism is operated in suction mode, the differential pressure is a negative pressure. In case the mechanism is operated in compressing mode, the differential pressure is a positive pressure. The pressure control system may include one or more pressure sensors to control the pressure level. Further, it may comprise actuators such as a valve or vent that adjusts the internal pressure. In applications involving contact with the skin, such as the electric device, the system facilitates to regulate fluid pressure, in particular air pressure, to ensure gentle, consistent contact without causing discomfort or injury.

As used herein, differential pressure compared to outside the sealed chamber, e.g. the ambient, refers to the difference in pressure between the sealed chamber, which is a closed or controlled environment, and the surrounding outside, i.e. atmospheric (ambient) pressure. It quantifies how much higher or lower the internal pressure is relative to the external environment. For example, if the sealed chamber maintains a pressure of 1200 hPa while the surrounding ambient pressure is 1000 hPa, the differential pressure is +200 hPa. Conversely, if the internal pressure is lower, such as 80 hPa, the differential pressure is -200 hPa. This facilitates where controlled pressure differentials are used for sealing, airflow control, or mechanical actuation-facilitating that the device operates reliably despite changes in ambient conditions.

According to the first aspect, the device comprises a release unit 400 configured to activate the optical system 120 or the electrode system 150 based on the differential pressure in the sealed chamber 300. For example, in case of the negative pressure, the device is attached without further means to the skin. In case of a positive pressure, further means such as a belt, may be used to attach the device to the skin. Positive pressure may facilitate further treatments.

As used herein, the release unit is a component processing an input, the differential pressure in the sealed chamber, to decide if the optical system is activated by an output. For example, the release unit is a circuitry receiving as input a sensor value from the pressure control system. This value may be compared with a preset threshold value stored in a memory. The output is for example a voltage value activating the optical system. This facilitates to ensure security by considering an input relating to a pressure value.

A second aspect relates to the electric device 10 according to aspect 1, wherein the optical system 120 comprises one or more light sources 122. A light source is a device for emitting photons. For example, as shown in Fig. 1, the system comprises a plurality of light sources. For example, at least one of the light sources 122 comprises at least one of a laser and a LED. Additionally or alternatively, the release unit 400 is configured to activate the one or more light sources 122. For example, based on an output of the release unit 400, the light source is powered.

A third aspect relates to the electric device 10 according to any one of the preceding aspects, the optical system 120 comprises one or more lenses 124. A lens is an optical component made from a transparent material-typically glass or plastic-that is shaped to converge or diverge light rays as they pass through it, thereby controlling the focus, direction, and distribution of light. Lenses are defined by their curvature, thickness, and refractive index, and can be categorized as either convex (converging) or concave (diverging) based on their shape. For example, as shown in Fig. 1, the outward-curved surfaces of the one or more lenses 124 protrude from the inner wall 112. This facilitates to focus light of the light source and to deflect tissue by the curvature of the lens. Additionally or alternatively, the one or more lenses 124 focus light in a distance of greater than or equal to 1 cm from the exit aperture 121. This facilities to treat tissue under the skin in a region 210. Additionally or alternatively, the one or more lenses 124 focus light in a distance of less than or equal to 5 cm from the exit aperture 121.

A fourth aspect relates to the electric device 10 according to any of the preceding aspects, wherein the exit aperture 121 comprises an outward-curved surface 121 that protrudes form the inner wall 110. This facilitates to deflect tissue by the curvature of the exit aperture as for example shown in Fig. 3. For example, a curvature of the exit aperture 122 is larger than a curvature of a flat portion of the support structure 110 surrounding the exit aperture. Thus, the flat portion can be closely arranged to the skin and tissue along the optical path can be deflected. Additionally or alternatively, the outward-curved surface protrudes form the inner wall 112 more than or equal to 1 mm. Additionally or alternatively, the outward-curved surface protrudes form the inner wall less than or equal to 5 mm.

A fifth aspect relates to the electric device 10 according to any of the preceding aspects, wherein the support structure 110 comprises a flat portion 114 for contacting the skin 200, wherein the exit aperture 121 is arranged in the flat portion. The flat portion facilitates to contact the skin. The flat portion may comprise a vibration element, e.g. a piezo element. For example, the flat portion 110 has a diameter of more than or equal to 5 cm. Additionally or alternatively, the flat portion 110 has a diameter of less than or equal to 15 cm. The flat portion 114 may comprise stainless steel. The flat portion may be heated, for example, the flat portion may comprise a resistor heating. Additionally or alternatively, the flat portion 114 may comprise a loudspeaker, for example one or more piezo elements, for generating sound, in particular ultrasonic sound with a wavelength of greater than or equal to 20 kHz to less than or equal to 10 MHz. In other words, the flat portion with the optical system 120 may vibrate, for example by using the piezo elements. This facilitates to increase blood circulation. For example, the flat portion may vibrate with a wavelength of greater than or equal to 10 kHz to less than or equal to 10 MHz. In particular, the flat portion may vibrate with a wavelength of greater than or equal to 100 kHz to less than or equal to 300 kHz Additionally or alternatively, the flat portion 114 may comprise an applicator for applying a RF or EMS signal. For example, the applicator on the flat portion may be a bipolar applicator as described above.

A sixth aspect relates to the electric device 10 according to any of the preceding aspects, wherein the pressure control system 140 comprises a vacuum source for generating a negative pressure.

A seventh aspect relates to the electric device 10 according to any of the preceding aspects, wherein the sealed chamber 300 comprises a recessed portion 116 formed in the support structure 110 as for example shown in Fig. 1. The recessed portion 116 is arranged between the exit aperture 121 and the sealing 130. The recessed portion refers to an indented area within the inner wall, deliberately formed to sit below the surrounding level of the inner wall and the sealing. The recessed portion 116 can be set to a pressure level with negative pressure, which facilitates to secure the device to the skin. In particular, the recessed portion 116 is surrounding the exit aperture 121.

An eighth aspect relates to the electric device 10 according to any of the preceding aspects, wherein a vacuum tube is connecting a recessed portion 116 formed in the support structure 110 and the pressure control system 140. A vacuum tube is a sealed, hollow structure that facilitates to remove fluids as air from the recessed portion 116.

A ninth aspect relates to the electric device 10 according to any of the preceding aspects, wherein the support structure 110 comprises an opaque material for blocking light emitted by the optical system 120. An opaque material is a substance that does not allow light to pass through it, meaning it completely blocks the transmission of for example visible, infrared, and/ or ultraviolet light. When light strikes the surface of an opaque material, it at least one of absorbed and reflected, but not transmitted, resulting in no visibility through the material. For example, the support structure 110 comprises an absorbing material for absorbing light emitted by the optical system.

A tenth aspect relates to the electric device 10 according to any of the preceding aspects, wherein the sealing 130 comprises an opaque material for blocking light emitted by the optical system. For example, the sealing 130 comprises an absorbing material for absorbing light emitted by the optical system.

An eleventh aspect relates to the electric device 10 according to any of the preceding aspects, wherein a light source 122 of the optical system 120 is configured to emit light in at least one of the red and infrared wavelength ranges. As used herein, the red wavelength range comprises light with a wavelength greater than and equal to 620 to less than and equal to 750 nm and infrared wavelength range comprises light with a wavelength greater than and equal to 750 to less than and equal to 1400 nm. Red and infrared light facilitates to heat the tissue.

A twelfth aspect relates to the electric device 10 according to any of the preceding aspects, wherein one or more light sources 122 of the optical system are configured to emit light with a power of equal to or greater than 0.1 W. Additionally, the one or more light sources 122 may be configured to emit light with a power of equal to or less than 5 W.

A thirteenth aspect relates to the electric device 10 according to any of the preceding aspects and further comprising a sensor to detect the differential pressure. For example, the sensor comprises a differential pressure sensor arranged in the sealed chamber 300. Additionally or alternatively, the sensor comprises a power sensor for sensing a power drawn by the pressure control system 140, in particular the mechanism such as the air pump to regulate the pressure level.

A fourteenth aspect relates to a method for controlling an electric device 10 for treatment of a skin 200, the method comprising the following steps:
providing a support structure 110 with an optical system 120 for illuminating the skin 120, wherein an exit aperture 121 of the optical system 120 is arranged on an inner wall 112 of the support structure 110;
providing a sealing 130 arranged on the support structure 110, the sealing 130 surrounding the exit aperture 121, wherein the sealing 130, when contacting the skin 200, forms a sealed chamber 300 comprising the inner wall 112;
activating a pressure control system 140, the pressure control system 140 being in fluid connection with the sealed chamber 300 for generating a differential pressure in the sealed chamber 300 compared to outside the sealed chamber;
activating by a release unit 400, based on a detected differential pressure in the sealed chamber 300, the optical system 120.

For the description of the components listed in the above method, reference is made to the above description of the device 10. In particular, the method may comprise the step of providing the device 10 according to any of the first through thirteenth aspects.

A fifteenth aspect relates to the method according to aspect 14, wherein the method further comprises:
staring a timer for counting a duration of the detected differential pressure, wherein the release unit 400 activates the optical system 120 upon expiry of the timer.

For example, the device may comprise a memory storing for example a threshold value and/or a timer value so that the light source is activated after the pressure has been stabilized.

A sixteenth aspect of the present disclosure relates to an electric device according to any of the above aspects for attachment to a skin. The device can be physically secured to the skin surface to maintain consistent contact during use. This facilitates effective and safe transmission of electrical energy (as in EMS or RF treatments), ensuring the device stays in the correct position and delivers energy evenly. In practice, attachment to the skin can be achieved through various means, including at least one of the following: adhesive surfaces (e.g. gel pads or sticky electrodes), straps, belts, or bands that hold device in place; suction or vacuum mechanisms; and conductive gels or pastes that improve electrical contact and help adhesion.

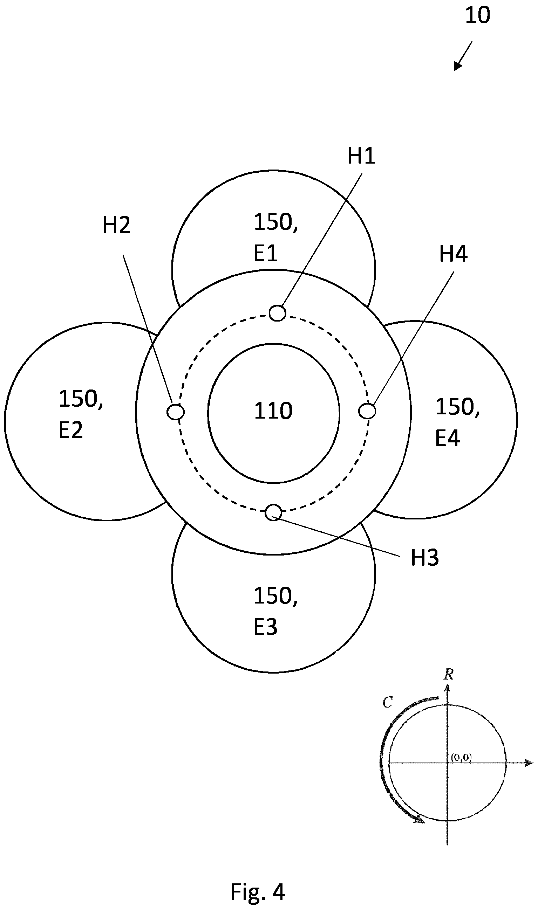

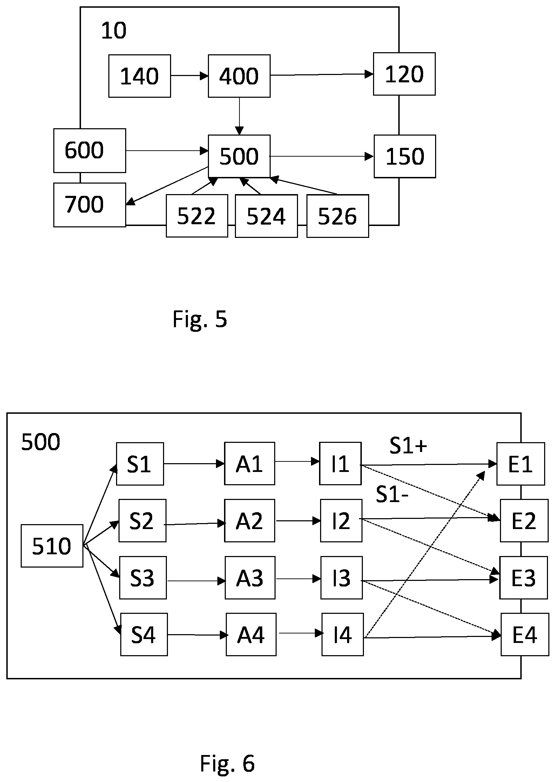

For example, as shown in Figs. 1 to 3, a vacuum mechanism 140 can be used. A top view of the electric device is shown in Fig. 4. A block diagram of the electric device 10 is shown in Fig. 5.

According to the sixteenth aspect the electric devices comprises a controller 500. A block diagram of the controller 500 is shown in Fig. 6.

The controller 500 comprises a signal generator 510 configured to generate four time dependent electrical signals S1, S2, S3, S4. For example, the controller comprises four signal generators for generating four time dependent symmetrical electrical signals S1, S2, S3, S4. Alternatively, the controller comprises a signal generator with four channels for generating four time dependent symmetrical electrical signals S1, S2, S3, S4. The signal generator 510 may be configured to generate m time dependent signal, with m being an integer greater than or equal 4.

As used herein, a signal generator is an electronic device that produces electrical waveforms, also referred to as signals, over at least one of a range of frequencies, amplitudes, and wave shapes. These signals are used primarily for being supplied to pads. The signal generators can produce various types of waveforms, such as sine waves, square waves, triangle waves, and pulses. These waveforms are examples of time dependent signals, where the signal is a function whose output (or value) varies as time progresses . For example, the signal S1= A*sin (w*t+ Φ), where ω is the frequency, A is amplitude, and Φ is a phase characteristics. The value of the waveform is adjustable in terms of frequency (Hz), amplitude (V), and phase. This facilitates to stimulate nerves or muscles safely or to heat tissue.

For example, the signal generators are embedded within the device 10 and configured to deliver timed and shaped electrical pulses. This facilitates a compact arrangement. In particular, the controller 500 is configured to adjust a component difference, for example a phase difference, a shape difference, a frequency difference, between different electrical signals S1, S2, S3, S4. This facilitates to generate an interference pattern. As an economic signal generator may be used a soundcard.

As used herein, the signal is for example a symmetric signal or a non-symmetric signal. In particular, each signal comprises two sub-signals for being supplied to a pair of pads for forming a bipolar applicator. As used herein, a symmetrical signal is an electrical waveform in which the positive and negative halves are mirror images in both shape and magnitude, typically centered around a zero-voltage baseline or a bias voltage. This means the waveform spends equal time and has equal amplitude in the positive and negative directions, creating a balanced, charge-neutral signal over time. For example, the positive signal S1+ may be a sine wave S1+= A1* sin (w*t +Φ) +A0 and the negative signal S1- may be shifted by half the period, e.g. S1- = A1* sin (w*t +Φ + π) +A0. This facilitates to reduce skin irritation, to improve comfort, and to avoid electrochemical damage to tissues. A non-symmetrical signal is an electrical waveform, which comprises only one of the halves, and the other is for example ground or a bias voltage.

According to the sixteenth aspect, the electric device comprises a one or more support structures 110, each support structure having an electrode system 150 for contacting the skin as for example shown in Figs. 1 to 4. The electrode system 150 comprises four pads E1, E2, E3, E4. The electrode system may comprise further electrodes, for example m *4 pads, with m being an integer greater than or equal 41.

As used herein a pad, in the context of electrical stimulation and therapeutic devices, refers to an electrode that can be flat and/or flexible. The pad is configured to attach to the skin and deliver electrical signals or energy (such as EMS, TENS, or RF). In other words, pads are the interface between the device and the skin of the body, facilitating effective and safe transmission of energy into the underlying tissues. The pad may have a conductive surface and is for example made of carbon rubber, silver-coated fabric, or metal mesh, to distribute electrical current evenly. The pads may come with a self-adhesive hydrogel layer that sticks to the skin and provides good electrical conductivity without irritation.

According to the sixteenth aspect, each of the electrical signals, in particular the symmetrical signal comprising halves S+ and S-, is applied to a pair of pads. Alternatively, the non-symmetrical signal comprising, for example one halve S+ and a bias voltage, is applied to a pair of pads. For example, as shown in Fig. 6, pads E1 and E2 are subject to the same signal S1 with halves S+ and S-. Thus, this arrangement facilitates that signal generator S1 and the pair of pads E1 and E2 form a first bipolar applicator. In total, the device comprises four bipolar applicators. Each of the four bipolar applicators facilitates to supply an electric field to a region 210 under the skin 200. Depending on the signal, different pairs of bipolar applicators may be controlled. In other words, the above described connection establishes a multipolar applicator controllable by the four signals.

As used herein, a bipolar applicator is a medical or surgical instrument that uses two for example closely spaced pads (e.g. a centroid of the pads has distance of greater than or equal to 2 cm and less than or equal to 20 cm) to deliver radiofrequency (RF) energy, electrical current, or thermal energy depending on a waveform generated by the signal generator to a region 210 under the skin 200, e.g. a targeted tissue area. Unlike monopolar devices (which use a separate grounding pad), bipolar applicators complete the electrical circuit entirely within the device, between the two electrodes, facilitating focusing energy precisely and locally.

According to the sixteenth aspect, the electrical device further comprises a circuitry 600 configured to instruct the controller 500 to adjust the component difference, e.g. the phase difference ΔΦ, between different electrical signals, e.g. between S1 and S2, between S2 and S3, between S3 and S4 and between S4 and S1. In other words, each of signals S1 to S4 follows a waveform, e.g. Sn= An1* sin (wn*t +Φn) +An0, where Φn = Φ1 to Φ4 with at least one of Φ1≠Φ2; Φ2≠Φ3; Φ3≠Φ4; and Φ4≠Φ1. The phase difference ΔΦ facilitates to control a set position of a superimposed field of the interfering electric fields in the region under the skin. In other words, the controlled phase difference facilitates constructive and destructive interference. Notably, further characteristics of the waveform may be additionally or alternatively adjusted.

An example is shown for example shown in Fig. 4. A hot spot in view of an RF signal can be moved from a first position at H1 to further positions H2 to H4, e.g. circulated along a circumferential direction.

According to a seventeenth aspect, the four pads according to the any of the above aspect are arranged along a circumferential direction C around the support structure 110. The circumferential direction refers to the direction along the circumference of a for example circular or cylindrical object, here the support structure, in particular it means around the object. This facilitates to activate tissue surrounded by the pads.

According to an eighteenth aspect, the signal generator according to any of the preceding aspects comprises a plurality of channels for generating four time dependent electrical signals each with a plurality of frequencies. This facilitates that the bipolar applicator can supply electric fields with different frequency to one region. Additionally or alternatively, the time dependent electrical signals have a frequency w or a plurality of frequencies w1 and w2 equal to or larger than 10 Hz and equal to or less than 10 MHz for treating a region below the skin. For example, the electrical signals have a frequency w1 of equal to or larger than 0.4 MHz and equal to or less than 10 MHz for heating the region. Additionally or alternatively, the electrical signals have a frequency w2 of equal to or larger than 10 Hz and equal to or less than 10 kHz for stimulating muscles in the region. In particular, the combination of w1 and w2 is advantageous.

According to a nineteenth aspect, the electrical signal according to any of the preceding aspects is a symmetrical electrical signal described above. For example, the symmetrical electrical signal comprises two complementary signals with a fixed phase difference, e.g. of Φ= 180°.

According to a twenties aspect, the support structure according to any of the preceding aspects further comprises a flat portion 114 for contacting the skin 200. This is in particular shown in Figs. 1 to 3. This flat portion 114 facilitates to integrate further devices to stimulate the skin. For example, the flat portion comprises optical or acoustic devices. Additionally or alternatively, the flat portion 114 facilitates to attach the device to the skin.

For example, the four pads E1 to E4 are surrounding the flat portion 114. The flat portion 114 may comprise an optical system 120 for illuminating the skin, wherein an exit aperture 121 of the optical system 120 is arranged on an inner wall 112 of the support structure 110. The flat portion 114 may comprise stainless steel. The flat portion may be heated, for example, the flat portion may comprise a resistor heating. Additionally or alternatively, the flat portion 114 may comprise a loudspeaker, for example one or more piezo elements, for generating sound, in particular ultrasonic sound with a wavelength of greater than or equal to 20 kHz to less than or equal to 10 MHz. In other words, the flat portion with the optical system 120 may vibrate, for example by using the piezo elements. This facilitates to increase blood circulation. For example, the flat portion may vibrate with a wavelength of greater than or equal to 10 kHz to less than or equal to 10 MHz. In particular, the flat portion may vibrate with a wavelength of greater than or equal to 100 kHz to less than or equal to 300 kHz Additionally or alternatively, the flat portion 114 may comprise an applicator for applying a RF or EMS signal. For example, the applicator on the flat portion may be a bipolar applicator as described above.

According to a further example, the electric device further comprises a sealing 130 arranged on the support structure 110, the sealing 130 surrounding the flat portion 114, wherein the sealing 130, when contacting the skin, forms a sealed chamber 300 comprising an inner wall 112 of the support structure 110.

A twenty-first aspect relates to the electric device according to any one of the preceding aspects and further comprises an intensity sensor 522. The intensity sensor facilitates sensing the intensity of the superimposed field. For example, the intensity sensor comprises a current sensor for sensing the current supplied to the pads by the electrical signals. Based on the sensed current and a set voltage for the signal, the power supplied to the tissue can be derived. Further, by the current distribution, an actual position of the superimposed field can be determined.

A twenty-second aspect relates to the electric device according to any one of the preceding aspects and further comprises an electric field sensor 524. The electric field sensor 524 facilitates sensing an actual position of the superimposed filed. An electric field sensor is a device used to detect and measure electric fields. For example, the support structure further comprises the above discussed flat portion 114 for contacting the skin 200 and the flat portion 114 comprises the electric field sensor. As discussed above, the four pads may surround the electric field sensor. There are several detection mechanisms for detecting electric fields. For example, a capacitive coupling may be used, which detects changes in capacitance caused by nearby charges. Further electro-optic effects may be used, which uses changes in optical properties of materials under an electric field. For example, MEMS-based sensors may be used, which are micro-electromechanical systems that respond to forces from electric fields.

A twenty-third aspect relates to the electric device according to any one of the preceding aspects and further comprises a temperature sensor 526 for sensing a temperature of the region under the skin. For example, at least one of the pads comprises the temperature sensor. In particular, all pads may comprise a temperature sensor. According to a further example, the four pads are arranged along a circumferential direction around the support structure and the temperature sensor is arranged between neighboring pads.

Any of the above discussed sensors may be used to modify the set position. In particular, the circuitry may be configured to control the set position based on the any of the above sensor values. In other words, the circuitry may comprise a feedback loop for controlling the set position based on the measured position measured by a sensor.

A twenty-fourth aspect relates to the electric device according to any one of the preceding aspects and the circuitry 600 comprises a user terminal for inputting, by a user, a command instructing the controller to adjust the phase difference between different symmetrical electrical signals for changing the set position of the superimposed field. For example, the user may select on a touch display to select a set position and the command may comprise the selected set position.

Additionally or alternatively, the command may be a serious of instruction, e.g. a circulation of the superimposed field. Additionally or alternatively, the command may be received wirelessly by the device, for example by a transmission protocol such as Bluetooth ^{®}.

A twenty-fifth aspect relates to the electric device according to any one of the preceding aspects and further comprises display output 700 outputting to a display information relating to the superimposed field. For example, display output may be a wireless interface for sending display information to a display. Additionally or alternatively, the device may comprise the display. The display information can comprise position information of the set position and/or the information relating to a sensed value. The display may be used for displaying the set position of the superimposed field relative to the electric device. Additionally or alternatively, the display may be used to display position derived by any of the above discussed sensors. Further, the display may be in particular used in combination with the above discussed user input. For example, the display may be a touch panel. Additionally or alternatively, the may be transmitted wirelessly by the device, for example by a transmission protocol such as Bluetooth ^{®}.

An twenty-sixth aspect relates to a method for controlling a set position of a superimposed field of interfering electric fields in a region under the skin. The method comprises the steps of:
providing an electric device according to any one of the preceding aspects; and
adjust a phase difference between different electrical signals to control the set position.

For example, the signals S1 to S4 may be A* sin ((w) *t). By modifying one of the signals, for example the phase of S3= A* sin (ω *t +Φ), the set position can be controlled.

According to a twenty-seventh aspect, the phase difference between different symmetrical electrical signals according the twenty-sixth aspect is changed based on a periodic function to move the set position of the superimposed field along a circumferential direction of the support structure. For example, the phase difference of the signal Sn+1 to signal Sn is Φn= 90°*(n-1), with n being an integer between 1 and 4. In other words, Φn is a periodic function. In this example, the waveforms may be for example S1= A* sin (w*t +0), S2= A*sin (w*t +90°), S3= A* sin (w*t +180°), and S4 = A* sin (w*t + 270°). This facilitates to rotate the set position. According to an alternative, a frequency difference of the signal Sn+2 to signal Sn may be Δw, with n being an integer between 1 and 2. In other words, the waveforms may be for example S1= A* sin (w*t +0), S2= A*sin (w*t +90°), S3= A* sin ((w+ Δw)*t +180°), and S4 = A* sin ((w+ Δw=*t + 270°). This facilitates to rotate a signal with frequency w with a frequency Δω.

According to a twenty-eight example, an amplitude of the different symmetrical electrical signals according to any of the twenty-sixth to twenty-seventh aspect is changed to move the set position of the superimposed field along a radial direction of the support structure. For example, an amplitude of the signal S1 and S3 may be A and an amplitude of the signal S2 and S2 may A/2 In other words, the waveforms may be for example S1= A* sin (w*t +0), S2= A/2*sin (w*t +90°), S3= A* sin ((w)*t +180°), and S4 = A/2* sin ((ω =*t + 270°). This facilitates to change for example a rotating set position discussed in the twelfth example in the radial direction.

According to a twenty-ninth aspect, a frequency of the electrical signals according to any of the twenty-sixth to twenty-eight aspect is aspect is changed to move the set position of the superimposed field relative along an axial direction (A) to the support structure. For example, the signals S1 to S4 may be A* sin ((w) *t). By modifying the frequency of one of the signals, for example S3= A* sin ((w- Δw) *t +Φ), the set position in depth of tissue, i.e. a distance to the skin, can be controlled.

According to a thirties aspect, set position set according to any of the twenty-sixth to twenty-nineth aspect is changed based on a user command input by a user. For example, the user command is input as described in the ninth aspect.

Variants of the present disclosure will now be described in detail with reference to FIGS. 1 through 6.

Fig. 1 shows an electric device 10 for attachment on a skin 200 of a human. The device 10 comprises a support structure 110 supporting task-performing components for performing tasks and control components to control the task performing components. The task performing components comprise an optical system 120, a sealing 130, a pressure control system 140, and an electrode system 150. The control components comprise a release unit 400 and a controller 500. Further, as shown with reference to Fig. 5, the support structure 110 can support communication components such as a circuitry 600 comprising a user terminal for inputting, by user, a command, and a display output 700 for outputting display information. Additionally or alternatively, the circuity the communication component may comprise a not shown input output unit. Further, the support structure 110 can comprise sensing components such as sensors 522, 524, and 526.

The support structure 110 mechanically supports the task performing components. Even not shown with reference to the Figures, the support structure 110 may support only one or a subgroup of the task performing components. Further, the task performing components may be fixed or removable connected to the support structure.

The support structure 110 may for example be a support plate with a cylindrical shape. As for example shown in Fig. 4, the base of the support structure 110 has a cylindrical surface. The base may have any other polygonal circumference, e.g. quadratic or rectangular. The support structure has two opposing faces, wherein a first face is in the following referred to as an inner wall 112 and the opposing second face is referred to as an outer wall, which may be in contact to the ambient. Further, the support structure 110 can comprise a circumferential face.

In particular, the support structure 110 comprises a flat portion 114 for contacting the skin 200. As shown in Fig. 1, the flat portion 114 is arranged on the inner wall 112. For example, the flat portion 114 has a diameter of more than or equal to 5 cm. Additionally or alternatively, the flat portion 114 has a diameter of less than or equal to 15 cm. The flat surface 114 may be flat compared to curved portions such as convex lens. The flat portion may comprise a material such as a plastic for contacting the skin. The support structure 110 may comprise an opaque material for blocking light emitted by the optical system 120. For example, the support structure 110 comprises an absorbing material for absorbing light emitted by the optical system.

The flat portion 114 may comprises an optical system 120 for illuminating the skin. Additionally or alternatively, the flat portion 114 may comprise a loud speaker, for example one or more piezo elements, for generating sound, in particular ultrasonic sound with a wavelength of greater than or equal to 20 kHz to less than or equal to 10 MHz. Additionally or alternatively, the flat portion 114 may comprise an applicator for applying a RF or EMS signal. For example, the applicator on the flat portion may be a bipolar applicator as described above. The combination of RF and optical systems may heat tissue. The activation by sound helps to vibrate the tissue thereby distributing the heat and/or EMS signals. Additionally or alternatively, the flat portion with the optical system 120 may vibrate, for example by using the piezo elements. This facilitates to increase blood circulation. For example, the flat portion may vibrate with a wavelength of greater than or equal to 10 kHz to less than or equal to 10 MHz. In particular, the flat portion may vibrate with a wavelength of greater than or equal to 100 kHz to less than or equal to 300 kHz

The optical system 120 supported by the support structure comprises one or more optical units. Each optical unit comprises a light source 122 and optionally a lens 124. As shown in the example of Fig. 1, the optical units are arranged in the support structure. Each optical unit has an exit aperture 121, which is arranged on the inner wall 112 of the support structure 110.

For example, the light sources 122 comprises a laser and/or an LED. The light source 122 of the optical system 120 may be configured to emit light in at least one of the red and infrared wavelength ranges. This facilitates to heat tissue. Additionally or alternatively, the light source 122 of the optical system may be configured to emit light with a power of equal to or greater than 0.1 W. Additionally or alternatively, the light source 122 of the optical system may be configured to emit light with a power of equal to or less than 5 W.

Further, an outward-curved surface of the lenses 124 protrude from the inner wall 112. For example, the lens 124 may focus light in a distance of greater than or equal to 1 cm from the exit aperture 121. Additionally or alternatively, the lens 124 may focus light in a distance of less than or equal to 5 cm from the exit aperture 121. Thus, deeper tissue may be treated. In other words, an exit aperture 121 of the optical system, which comprises an outward-curved surface 121, protrudes form the inner wall 110. In particular, a curvature of the exit aperture 122 is larger than a curvature of a flat portion of the support structure 110 surrounding the exit aperture. Additionally or alternatively, the outward-curved surface protrudes form the inner wall 112 more than or equal to 1 mm. Additionally or alternatively, the outward-curved surface protrudes form the inner wall less than or equal to 5 mm.

The sealing 130 may be arranged on the support structure 110. The sealing 130 surrounds the flat portion 114. In particular, the sealing 130 surrounds the exit aperture 121. As shown in Fig. 2, when the sealing 130 contacts the skin 200, a sealed chamber 300 comprising the inner wall 112 of the support structure 110, is formed. The sealing 130 may comprise an opaque material for blocking light emitted by the optical system 120. For example, the sealing 130 comprises an absorbing material for absorbing light emitted by the optical system.

The sealed chamber 300 may comprise a recessed portion 116 formed in the support structure 110. The recessed portion 116 is for example arranged between the exit aperture 121 and the sealing 130. For example, the recessed portion 116 is surrounding the exit aperture 121.

The device may further comprise the pressure control system 140. The pressure control system 140 is in fluid connection with the sealed chamber 300 for generating a differential pressure in the sealed chamber 300 compared to outside the sealed chamber. For example, the pressure control system 140 comprises a vacuum source for generating a negative pressure in the sealed chamber. In particular, a vacuum tube connects the recessed portion 116 formed in the support structure 110 with the pressure control system 140.

The device 10 may further comprise a sensor to detect the differential pressure. For example, the sensor comprises a differential pressure sensor arranged in the sealed chamber 300. Additionally or alternatively, the sensor comprises a power sensor for sensing a power drawn by the pressure control system 140. Thus, the differential pressure in the sealed chamber 300 relative to the exterior ambient pressure can be determined.

The device 10 may further comprise a controller 500 comprising a signal generator 510 configured to generate four time dependent electrical signals S1, S2, S3, S4. In particular, the controller 500 is configured to adjust a phase difference between different electrical signals S1, S2, S3, S4. The controller may additionally or alternatively be configured to control the optical system.

Further, the support structure 110 may comprise an electrode system 150 for contacting the skin. The electrode system 150 comprises, as for example shown in Fig. 4, four pads E1, E2, E3, E4. The pads are arranged at circumferential face of the support structure 110. As for example shown in Fig. 6, each of the electrical signals S1+, S1-; S2+, S2-; S3+, S3-; S4+, S4- is applied to a pair of pads E1, E2; E2, E3; E3, E4; E4, E1; to form a four bipolar applicators supplying an electric field to a region 210 under the skin 200. The configuration facilitates to mix electrical signals that are applied to the four pads.

Even not shown in Fig. 6, the signal generator 510 may comprise a plurality of channels for generating the four time dependent electrical signals each with a plurality of frequencies.

As shown in Fig. 6, the controller 500 may comprise four signal generators S1 to S4. Each of the signal generators S1 to S4 may be connected to further circuitries such as amplifiers A1 to A4 and isolators I1 to I4.

The time dependent electrical signals may have a frequency of equal to or larger than 10 Hz. Additionally or alternatively, the frequency may be equal to or less than 10 MHz for treating a region below the skin. For example, the electrical signals have a frequency of equal to or larger than 0.4 MHz. Additionally or alternatively, the electrical signals may have a frequency of equal to or less than 10 MHz for heating the region. Additionally or alternatively, the electrical signals may have a frequency of equal to or larger than 10 Hz. Additionally or alternatively, the electrical signals may have a frequency of equal to or less than 10 kHz for stimulating muscles in the region. According to one example, the electrical signal is a symmetrical electrical signal comprising two complementary signals with a fixed phase difference. For example, S1+= A* sin (ω *t) and S1-= A* sin (ω *t +180°).

As for example shown in Fig. 4, the four pads E1 to E4 are arranged along a circumferential direction C around the support structure 110. In particular, the support structure may comprise the above described flat portion 114 for contacting the skin 200 and the four pads are surrounding the flat portion 114.

Further, the electric device 10 comprises a circuitry 600 configured to instruct the controller 500 to adjust the phase difference between different electrical signals S1, S2. This facilitates to control a set position of a superimposed field of the interfering electric fields in the region under the skin.

This facilitates to move the set position of the superimposed field along a circumferential direction of the support structure. For example, the signals S1 to S4 may be A* sin ((w) *t). By modifying one of the signals, for example the phase of S3= A* sin (ω *t +Φ), the set position can be controlled.

In particular, the phase difference between different symmetrical electrical signals according is changed based on a periodic function to move the set position of the superimposed field along a circumferential direction C of the support structure. For example, the phase difference of the signal Sn+1 to signal Sn is Φn= 90°*(n-1), with n being an integer between 1 and 4. In other words, Φn is a periodic function. In this example, the waveforms may be for example S1= A* sin (w*t +0), S2= A*sin (w*t +90°), S3= A* sin (w*t +180°), and S4 = A* sin (w*t + 270°). This facilitates to rotate the set position from H1 to H4 in Fig. 5. According to an alternative, a frequency difference of the signal Sn+2 to signal Sn may be Δw, with n being an integer between 1 and 2. In other words, the waveforms may be for example S1= A* sin (w*t +0), S2= A*sin (w*t +90°), S3= A* sin ((w+ Δw)*t +180°), and S4 = A* sin ((w+ Δw=*t + 270°). This facilitates to rotate a signal with frequency w with a frequency Δw. Additionally or alternatively, an amplitude of the different symmetrical electrical signals can be changed to move the set position of the superimposed field along a radial direction of the support structure. For example, an amplitude of the signal S1 and S3 may be A and an amplitude of the signal S2 and S2 may A/2 In other words, the waveforms may be for example S1= A* sin (w*t +0), S2= A/2*sin (w*t +90°), S3= A* sin ((w)*t +180°), and S4 = A/2* sin ((ω =*t + 270°). This facilitates to change for example a rotating set position discussed above in the radial direction. Additionally or alternatively, a frequency of the electrical signals according may be changed to move the set position of the superimposed field relative along an axial direction (A) to the support structure 110. For example, the signals S1 to S4 may be A* sin ((w) *t). By modifying the frequency of one of the signals, for example S3= A* sin ((w- Δw) *t +Φ), the set position in depth of tissue, i.e. a distance to the skin, can be controlled.

As for example shown in Fig. 6, the electric device 10 may further comprise at least one of sensors 522, 524, 526. For example, the device 10 may comprise an intensity sensor 522 for sensing the intensity of the superimposed field. For example, the intensity sensor comprises a current sensor for sensing the current supplied to the pads by the electrical signals. Additionally or alternatively, the electric device 10 may comprise an electric field sensor 524 for sensing an actual position of the superimposed electric field, e.g. the position H1 to H4. For example, the flat portion 114 for contacting the skin 200 comprises the electric field sensor. In particular, the four pads are surrounding the electric field sensor.

Additionally or alternatively, the device may comprise a temperature sensor 526 for sensing a temperature of the region under the skin. For example, at least one of the pads comprises the temperature sensor. Additionally or alternatively, the four pads are arranged along a circumferential direction C around the support structure 110 and the temperature sensor is arranged between neighboring pads.

As discussed above, the electric device 10 may comprise a communication circuitry. For example, the circuitry 600 may comprise a user terminal for inputting, by a user, a command. The command may for example for instructing the controller to adjust the phase difference between different electrical signals for changing the set position of the superimposed field. For example, the set position may be input on a touch panel showing the current set position and a user may select a new set position. Additionally or alternatively, the control may be any one a plurality of preset moving profiles, e.g. circulating the superimpose field. Additionally or alternatively, the command may for example for activating the pressure control system 140.

The application of the device to the skin is for example shown in the sequence of Figs. 1 to 3. In particular, upon arrangement of the device 10 to the skin 200, as shown in Fig. 2, a user may active the pressure control system 140. In particular, the sealed chamber 300 may experience a negative pressure. In view of the negative pressure, as shown in Fig. 3, the device 10 comes in close connection to the skin 200. In particular, the exit aperture 121 of the optical system may deflect tissue. Further, the electrode system 150 may come in close connection to the skin.

The device 10 may further comprise a release unit 400. The release unit 400 is configured to activate any of the optical system 120 and the electrode system 150 based on the differential pressure in the sealed chamber 300. For example, upon detecting that the pressure in the chamber 300 is different compared to the external, a timer may be started. The timer may count a duration of the detected differential pressure. The release unit 400 may then activate the optical system 120 and/or the electronic system 150 upon expiry of the timer.

As discussed above, the electric device 10 may comprise the communication circuitry. For example, the circuitry 600 may comprise a display output 700 outputting to a display information. For example, the display information may relate to the superimposed field. Additionally or alternatively, the display information may comprise the information that the release unit 400 activates any of the optical system 120 and the electrode system 150.

Even not shown in the Figures, the above discussed device 10 may be used in a method for controlling an electric device 10 for treatment of a skin 200. The method comprise the following the step of providing the support structure 110 with the optical system 120 for illuminating the skin 120, wherein an exit aperture 121 of the optical system 120 is arranged on an inner wall 112 of the support structure 110. Further, the method may comprise the step of providing a sealing 130 arranged on the support structure 110, the sealing 130 surrounding the exit aperture 121, wherein the sealing 130, when contacting the skin 200, forms a sealed chamber 300 comprising the inner wall 112. Then, the method may comprise the step of activating a pressure control system 140, the pressure control system 140 being in fluid connection with the sealed chamber 300 for generating a differential pressure in the sealed chamber 300 compared to outside the sealed chamber. Based on a detected differential pressure in the sealed chamber 300, the release unit 400 may activate the optical system 120 and/or the electrode system.

According to a further method, which can be provided as alternative or in addition, a set position 210 of the superimposed field of interfering electric fields in a region under the skin is controlled. In particular, the method comprises the steps of providing an electric device 10 with the electrode system as described above. Further, for example based on a user command input by a user, a phase difference between different electrical signals is adjusted to control the set position.

Even not shown with the Figures, a plurality of devices 10 may be provided. The plurality of devices and be controlled by one master control unit.

## Claims

1. Electric device (10) for attachment on a skin (200), the device (10) comprising:
a support structure (110) with an optical system (120) for illuminating the skin, wherein an exit aperture (121) of the optical system (120) is arranged on an inner wall (112) of the support structure (110);
a sealing (130) arranged on the support structure (110), the sealing (130) surrounding the exit aperture, wherein the sealing (130), when contacting the skin, forms a sealed chamber (300) comprising the inner wall (112);
a pressure control system (140) in fluid connection with the sealed chamber (300) for generating a differential pressure in the sealed chamber (300) compared to outside the sealed chamber; and
a release unit (400) configured to activate the optical system (120) based on the differential pressure in the sealed chamber (300).

2. Electric device (10) according to claim 1, wherein the optical system (120) comprises one or more light sources (122),
optionally wherein the one or more light sources (122) comprise a laser,
optionally wherein the release unit (400) is configured to activate the one or more light sources (122).

3. Electric device (10) according to any one of the preceding claims, wherein the optical system (120) comprises one or more lenses (124);
optionally wherein outward-curved surfaces of the one or more lenses (124) protrude from the inner wall (112);
optionally wherein the one or more lenses (124) focus light in a distance of greater than or equal to 1 cm from the exit aperture (121);
optionally wherein the one or more lenses (124) focus light in a distance of less than or equal to 5 cm from the exit aperture (121).

4. Electric device (10) according to any of the preceding claims, wherein the exit aperture (121) comprises an outward-curved surface (121) that protrudes form the inner wall (110), optionally wherein a curvature of the exit aperture (122) is larger than a curvature a flat portion of the support structure (110) surrounding the exit aperture;
optionally the outward-curved surface protrudes form the inner wall (112) more than or equal to 1 mm;
optionally the outward-curved surface protrudes form the inner wall less than or equal to 5 mm.

5. Electric device (10) according to any of the preceding claims, wherein the support structure (110) comprises a flat portion (114) for contacting the skin (200), wherein the exit aperture (121) is arranged in the flat portion, wherein the flat portion comprises a vibration element for vibrating the flat portion;
optionally the flat portion (114) has a diameter of more than or equal to 5 cm;
optionally the flat portion (114) has a diameter of less than or equal to 15 cm.

6. Electric device (10) according to any of the preceding claims, wherein the pressure control system (140) comprises a vacuum source for generating a negative pressure.

7. Electric device (10) according to any of the preceding claims, wherein the sealed chamber (300) comprises a recessed portion (116) formed in the support structure (110), the recessed portion (116) arranged between the exit aperture (121) and the sealing (130);
optionally wherein the recessed portion (116) is surrounding the exit aperture (121).

8. Electric device (10) according to any of the preceding claims, wherein a vacuum tube is connecting a recessed portion (116) formed in the support structure (110) and the pressure control system (140).

9. Electric device (10) according to any of the preceding claims, wherein the support structure (110) comprises an opaque material for blocking light emitted by the optical system (120);
optionally wherein the support structure (110) comprises an absorbing material for absorbing light emitted by the optical system.

10. Electric device (10) according to any of the preceding claims, wherein the sealing (130) comprises an opaque material for blocking light emitted by the optical system;
optionally wherein the sealing (130) comprises an absorbing material for absorbing light emitted by the optical system.

11. Electric device (10) according to any of the preceding claims, wherein a light source (122) of the optical system (120) is configured to emit light in at least one of the red and infrared wavelength ranges.

12. Electric device (10) according to any of the preceding claims, wherein one or more light sources (122) of the optical system are configured to emit light with a power of equal to or greater than 0.1 W;
optionally wherein the one or more light sources (122) are configured to emit light with a power of equal to or less than 5 W.

13. Electric device (10) according to any of the preceding claims further comprising a sensor to detect the differential pressure;
optionally wherein the sensor comprises a differential pressure sensor arranged in the sealed chamber (300);
optionally wherein the sensor comprises a power sensor for sensing a power drawn by the pressure control system (140).

14. Method for controlling an electric device (10) for treatment of a skin (200), the method comprising the following steps:
providing a support structure (110) with an optical system (120) for illuminating the skin (120), wherein an exit aperture (121) of the optical system (120) is arranged on an inner wall (112) of the support structure (110);
providing a sealing (130) arranged on the support structure (110), the sealing (130) surrounding the exit aperture (121), wherein the sealing (130), when contacting the skin (200), forms a sealed chamber (300) comprising the inner wall (112) ;
activating a pressure control system (140), the pressure control system (140) being in fluid connection with the sealed chamber (300) for generating a differential pressure in the sealed chamber (300) compared to outside the sealed chamber;
activating by a release unit (400), based on a detected differential pressure in the sealed chamber (300), the optical system (120).

15. Method according to claim 14, wherein the method comprises:
staring a timer for counting a duration of the detected differential pressure, wherein the release unit (400) activates the optical system (120) upon expiry of the timer.
